# EUROPÄISCHE PATENTSCHRIFT

(11) **EP 4 197 967 B1**
(45) Veröffentlichungstag und Bekanntmachung des Hinweises auf die Patenterteilung: **09.10.2024**
(21) Anmeldenummer: 21020646.2
(22) Anmeldetag: 20.12.2021
(51) Int. Cl.: C01B 3/36, C01B 3/38, C01B 3/52, C01B 32/40, C07C 29/151, C07C 31/04, B01J 8/00

(54) **VERFAHREN UND ANLAGE ZUM HERSTELLEN VON METHANOL UND KOHLENMONOXID**
METHOD AND INSTALLATION FOR PRODUCING METHANOL AND CARBON MONOXIDE
PROCÉDÉ ET INSTALLATION DE PRODUCTION DE MÉTHANOL ET DE MONOXYDE DE CARBONE

(43) Veröffentlichungstag der Anmeldung: 21.06.2023
(73) Patentinhaber: L'Air Liquide, Société Anonyme pour l'Etude et l'Exploitation des Procédés Georges Claude, 75007 Paris (FR)
(72) Erfinder: Weigand, Peter, D-60439 Frankfurt am Main (DE); Lim, Chin Han, D-60439 Frankfurt am Main (DE); Gronemann, Veronika, D-60439 Frankfurt am Main (DE); Stein, Matthias, D-60439 Frankfurt am Main (DE)
(74) Vertreter: Air Liquide

(56) Entgegenhaltungen:
- DE-B4- 10 214 003

## Beschreibung

### Gebiet der Erfindung

Die Erfindung betrifft ein Verfahren zum Herstellen von Methanol und reinem Kohlenmonoxid aus einem Kohlenwasserstoffe enthaltenden Einsatzstrom. Insbesondere betrifft die vorliegende Erfindung ein Verfahren zur parallelen Bereitstellung von Synthesegas für die Herstellung von Methanol und reinem Kohlenmonoxid unter Verwendung von gasförmigem oder flüssigem kohlenstoffhaltigem Einsatzmaterial wie bevorzugt Erdgas, aber auch schweren Raffinerierückständen und vergleichbaren kohlenstoffhaltigen Reststoffen in einem partiellen Oxidationsverfahren. Die Erfindung betrifft ferner eine Anlage zur Durchführung eines solchen Herstellungsverfahrens.

### Stand der Technik

Verfahren zur industriellen Herstellung von Methanol durch heterogen-katalytische Umsetzung von Synthesegas bzw. des hierin enthaltenen Wasserstoffs in geeigneten Synthesereaktoren sind der Fachwelt seit langem bekannt. Als Synthesegase bezeichnet man dabei Wasserstoff und Kohlenoxide enthaltende Gasgemische, die in verschiedenen Synthesereaktionen Verwendung finden.

Methanol stellt eine wichtige, unverzichtbare Basischemikalie der chemischen Industrie für die Weiterverarbeitung zu Endprodukten dar. In Ullmann's Encyclopedia of Industrial Chemistry, Sixth Edition, 1998 Electronic Release, Kapitel "Methanol", Unterkapitel 5 "Process Technology" uwerden verschiedene Grundverfahren zur Herstellung von Methanol beschrieben.

Ein moderneres, zweistufiges Verfahren zur Herstellung von Methanol ist beispielsweise aus der europäischen Patentschrift EP 0 790 226 B1 bekannt. Das Methanol wird in einem Kreisprozess hergestellt, bei dem eine Mischung von frischem und teilweise reagiertem Synthesegas zunächst einem wassergekühlten Reaktor (WCR) und dann einem gasgekühlten Reaktor (GCR) zugeführt wird, in welchen das Synthesegas jeweils an einem kupferbasierten Festbett-Katalysator zu Methanol umgesetzt wird. Das in dem Prozess hergestellte Methanol wird von dem zurückzuführenden Synthesegas abgeschieden, welches dann als Kühlmittel im Gegenstrom durch den gasgekühlten Reaktor hindurchgeführt und auf eine Temperatur von 220 bis 280 °C vorgewärmt wird, bevor es in den ersten Synthesereaktor eingebracht wird. Ein Teil des zurückzuführenden Synthesegases wird als Spülstrom (sog. Purge) aus dem Verfahren entfernt, um zu verhindern, dass sich Inertkomponenten innerhalb des Synthesekreislaufes anreichern.

Als Inertkomponente im Sinne der Methanolsynthese wird dabei nicht umgesetztes Methan aus der Synthesegaserzeugung betrachtet, da sich dieses unter den Bedingungen der Methanol- bzw. Ammoniaksynthese ebenfalls nicht weiter umsetzt. Dasselbe gilt für Argon, das über Feedströme in die Synthesegaserzeugung gelangt.

Es existieren unterschiedliche Verfahren zur Herstellung von Wasserstoff (H₂) und Kohlenoxide wie Kohlenmonoxid (CO) und Kohlendioxid (CO₂) umfassendem Synthesegas als Einsatzgas für die Methanolsynthese, beispielsweise die Dampfreformierung, die autotherme Reformierung (ATR), Kombinationen beider (sog. Combined Reforming) und die nichtkatalytische Partialoxidation (POX). Technische Einzelheiten dieser Verfahren sind der Fachwelt bekannt und werden beispielsweise in Ullmann's Encyclopedia of Industrial Chemistry, Sixth Edition, 1998 Electronic Release, Stichwort "Gas Production", im Detail erläutert. Im weiteren Sinne und für die Zwecke der vorliegenden Offenbarung wird dabei auch die autotherme Reformierung wegen des verwendeten Suaerstoffunterschusses gegeüber einer Totaloxidation bzw. vollständigen Verbrennung als partielles Oxidationsverfahren verstanden.

Als Ausgangsstoffe der oben genannten Verfahren zur Synthesegaserzeugung dienen dabei Kohlenwasserstoffe wie Erdgas mit seiner Hauptkomponente Methan oder Naphtha. Die genannten Verfahren liefern unterschiedliche Verhältnisse der Produktkomponenten Kohlenmonoxid (CO) und Wasserstoff (H₂), wie anhand der folgenden Reaktionsgleichungen ersichtlich ist:

| | | |
|---|---|---|
| 2 CH₄ + O₂ | = 2 CO + 4 H₂ | (Partialoxidation) |
| 2 CH₄ + ½ O₂ + H₂O | = 2 CO + 5 H₂ | (autotherme Reformierung) |
| 2 CH₄ + 2 H₂O | = 2 CO + 6 H₂ | (reine Dampfreformierung) |

Da bei der Partialoxidation oder der autothermen Reformierung mit einem Überschuss an Kohlenwasserstoff bzw. Unterschuss an Sauerstoff gearbeitet wird, um die Totaloxidation des Kohlenwasserstoffs zu Kohlendioxid zu unterdrücken, wird oft ein Synthesegas erhalten, dass in Bezug auf seine Verwendung als Einsatzgas für die Methanolsynthese ein Wasserstoffdefizit aufweist. Diese erfordert gemäß folgender Reaktionsgleichung

2 H₂ + CO = CHsOH

ein H₂/CO-Verhältnis von mindestens 2, unter praktischen Synthesebedingungen oft sogar etwas größer als 2, beispielsweise 2,1. Dieses Verhältnis wird üblicherweise als Stöchiometriezahl SN der Methanolsynthese formuliert, die berücksichtigt, dass auch Kohlendioxid zu Methanol reagiert:

SN = ([H₂] - [CO₂]) / ([CO] + [CO₂]) ≥ 2 (z. B. 2,1)

Mittels Partialoxidation oder autothermer Reformierung erhaltene Synthesegase weisen dagegen oft eine Stöchiometriezahl von ≤ 1,9, gelegentlich sogar ≤ 1,7 auf. Somit wird von keinem der Reformierungs- bzw. Partialoxidationsverfahren für sich betrachtet ein Synthesegasprodukt mit dem für die Methanolsynthese gewünschten, stöchiometrischen H₂/CO-Verhältnis von 2 bzw. mit nur leichtem Wasserstoff-Überschuss erhalten.

Wenn die Ausbeute an Wasserstoff auf Kosten des Kohlenmonoxids maximiert werden soll, ist es üblich, das Rohsynthesegas einer CO-Konvertierungsreaktion zu unterziehen, die auch als Wassergas-Shift-Reaktion (WGS) oder CO-Shift-Reaktion bezeichnet wird und gemäß nachfolgender Umsatzgleichung verläuft:

CO + H₂O = CO₂ + H₂

Die weitere Aufbereitung des erzeugten Rohsynthesegases umfasst zumeist auch ein Sorptionsverfahren zur Abtrennung weiterer unerwünschter Begleitstoffe, beispielsweise mittels physikalischer oder chemischer Absorption oder Gaswäsche. So können mit solchen Verfahren unerwünschte Bestandteile, insbesondere Kohlendioxid (CO₂), von den erwünschten Synthesegas-Hauptbestandteilen Wasserstoff und Kohlenmonoxid sicher bis in den Spurenbereich entfernt werden. Ein bekanntes und häufig angewendetes Verfahren ist das Rectisol-Verfahren, das eine Wäsche des Rohsynthesegases mit tiefkaltem Methanol als Absorbens umfasst und ebenfalls im oben genannten Schrifttum grundsätzlich beschrieben wird.

Zur Entfernung von Spuren höherer Kohlenwasserstoffe oder von Kohlenmonoxid kann die kryogene Gaszerlegung (sog. Coldbox) eingesetzt werden. Bei dieser wird hauptsächlich flüssiges Methan oder flüssiger Stickstoff verwendet, um höher siedende Gase wie Kohlenmonoxid zu absorbieren. Hierbei erhaltene Abgasströme können als Heizgas verwendet werden oder alternativ mittels weiterer kryogener Gaszerlegung in einen methanreichen Gasstrom und in einen weiteren, Kohlenmonoxid und Wasserstoff umfassenden Gasstrom aufgetrennt werden, falls dies erwünscht oder erforderlich ist. Weitere Einzelheiten zu Verfahren der kryogenen Gaszerlegung können dem Schrifttum entnommen werden, exemplarisch wird hierzu auf beispielsweise auf das Fachbuch Häring, H. W., Industrial Gases Processing, WILEY-VCH Verlag, Weinheim (2008), Chapter 5.2.3.6 "Cryogenic Separation Processes" verwiesen.

Produktionsverfahren für die parallele Herstellung von Methanol und Kohlenmonoxid sind aus dem Stand der Technik an sich bereits bekannt. Das Patent US 6,232,352 B1 beschreibt ein Verfahren zur gleichzeitigen Erzeugung von CO und Methanol für die Herstellung von Essigsäure durch Dampfreformierung. Bei diesem Verfahren wird durch die Befeuerung des Dampfreformers und des befeuerten Erhitzers für die Dampferzeugung eine große Menge an Kohlendioxid an die Atmosphäre abgegeben. Die CO₂-Emission kann dabei mehr als 5 kg CO₂ pro kg Methanol-Produkt betragen).

Das Patent DE 10214003 B4 beschreibt ein Verfahren zur Co-Produktion von Kohlenmonoxid und Methanol mit geringer oder ggf. sogar ohne CO₂-Emission durch katalytische oder nicht-katalytische partielle Oxidation eines gasförmigen oder flüssigen Einsatzstoffes unter Verwendung von Sauerstoff und Wasserdampf, wobei ein Teil des erzeugten Synthesegases abgespalten und das in diesem Gas enthaltene Kohlendioxid durch eine Gaswäsche abgetrennt, rekomprimiert und über den Feed-Injektor oder eine ähnliche Vorrichtung in den Synthesegasreaktor zurückgeführt wird.

### Nachteile des Stands der Technik

Bei den bisher bekannten Verfahren zur Herstellung von Synthesegas für Methanol und zur Herstellung von CO durch Dampfreformierung stammt die Wärme für den Reformierungsprozess aus der Verbrennung fossiler Brennstoffe, in der Regel Erdgas , bei der eine erhebliche Menge an CO₂ freigesetzt wird. Die Abscheidung dieses CO₂ mittels Abtrennung und Speicherung, z. B. Untertage-Speicherung (Carbon Capture and Storage, CCS) ist grundsätzlich möglich, erfordert aber einen erheblichen technischen und energetischen Aufwand sowie einen Bestimmungsort für die Speicherung und einen Transport dorthin.

In der oben beschriebenen Patentschrift DE 10214003 B4 zur Co-Produktion von CO und Methanol wird das erzeugte CO₂ mindestens teilweise zu der Partialoxidationsstufe zurückgeführt, was zu einer geringen CO₂-Emission führt. Allerdings ist bei diesem Verfahren mindestens die Verdichtung zweier CO₂-haltiger Prozessströme erforderlich. Darüber hinaus wird ein Teil des dem Partialoxidationsreaktor zugeführten CO₂ in CO umgewandelt, was in einigen Fällen von Nachteil ist, da bekannt ist, dass für eine effiziente Umwandlung bei der Methanolsynthese eine Stöchiometriezahl von etwa 2 eingestellt werden sollte, wobei Werte zwischen 2,0 und 2,2 als optimal betrachtet werden. CO reduziert die Stöchiometriezahl gemäß der oben angegebenen Berechnungsformel. Insbesondere bei Anwendung einer nicht-katalytischen partiellen Oxidation (POX) ist der CO₂ -Gehalt im Synthesegas in der Regel zu niedrig für ein optimales Einsatzgas für die Methanolsynthese. Generell werden bei einem nicht katalytischen Verfahren niedrigere CO₂ -Gehalte des Synthesegases (typisch 2 bis 4 Vol.-%) erhalten als bei Anwendung eines katalytischen Verfahrens (typisch 6 bis 8 Vol.-%, Vol.-%-Werte jeweils auf Trockenbasis am Ausgang des Reaktors). Dieses Synthesegas wird in der Regel am Reaktoreingang des Methanolsynthesereaktors mit Recyclinggas aus dem Synthesegaskreislauf der Methanolsynthese verdünnt.

Bei dem in der oben genannten Patentveröffentlichung beschriebenen POX-Verfahren mit Erdgas als Einsatzgas wäre daher der aus der Partialoxidation resultierende CO₂-Gehalt am Reaktoreingang des Methanolsynthesereaktors zu niedrig, was zu einer deutlich geringeren Effizienz des Methanolsynthese führen würde.

### Beschreibung der Erfindung

Die Aufgabe der vorliegenden Erfindung besteht daher darin, ein Verfahren und eine Anlage anzugeben, das die beschriebenen Nachteile des Stands der Technik nicht aufweist und das es insbesondere ermöglicht, in einem Verfahren zur parallelen Erzeugung von Methanol und Rein-Kohlenmonoxid möglichst alle anfallenden Stoffströme stofflich und/oder energetisch zu nutzen. Ferner soll mit der Erfindung eine optimale Einstellung der Stöchiometriezahl für die Methanolsynthese ohne Import von nicht im Verfahren erzeugtem Wasserstoff ermöglicht werden.

Diese Aufgabe wird in einem ersten Aspekt der Erfindung durch ein Verfahren mit den Merkmalen des Anspruchs 1 und durch eine Anlage mit den Merkmalen des Anspruchs 12 gelöst. Weitere Ausgestaltungen nach weiteren Aspekten der Erfindung ergeben sich aus den Unteransprüchen der jeweiligen Kategorie.

Unter Partialoxidationsbedingungen oder Methanolsynthesebedingungen sind die dem Fachmann an sich bekannten Verfahrensbedingungen, insbesondere von Temperatur, Druck, Verweilzeit, zu verstehen, wie sie oben beispielhaft genannt und detailliert im einschlägigen Schrifttum, beispielsweise der Patentschrift DE 10214003 B4, erörtert werden und bei denen mindestens ein Teilumsatz, bevorzugt allerdings technisch relevante Umsätze der Edukte zu den Produkten des jeweiligen Verfahrens erfolgt. Dasselbe gilt für die Auswahl eines geeigneten Katalysators im Falle der Methanolsynthese bzw. des autothermen Reformierens. Entsprechende Partialoxidationsreaktoren bzw. Methanolsynthesereaktoren sind der Fachwelt an sich bekannt und beispielsweise in dem eingangs erwähnten Schrifttum beschrieben.

Unter einer Sorptionsvorrichtung wird im Rahmen der vorliegenden Offenbarung eine Vorrichtung verstanden, die es ermöglicht, dass ein Fluidgemisch, beispielsweise ein Gasgemisch, mittels eines physikalischen oder chemischen Sorptionsverfahrens unter Verwendung eines geeigneten Sorbens in seine Bestandteile aufgetrennt bzw. unerwünschte Komponenten aus diesem Gemisch abgetrennt werden können. Das Sorptionsverfahren kann dabei auf einer Adsorption, also einer Bindung des oder der abzutrennenden Stoffe an eine Oberfläche oder Grenzfläche des festen Adsorbens, oder auf einer Absorption, also einer Aufnahme des oder der abzutrennenden Stoffe in das Volumen des flüssigen oder festen Absorbens, beruhen. Der oder die abgetrennten und mittels Sorption gebundenen Stoffe werden als Ad- bzw. Absorbat bezeichnet. Die dabei wirkenden Bindungskräfte können physikalischer oder chemischer Art sein. Entsprechend wirken bei der physikalischen Sorption zumeist schwächere, unspezifischere Bindungskräfte, z. B. van-der-Waals-Kräfte, wohingegen bei der chemischen Sorption stärkere, spezifischere Bindungskräfte wirken und das Ad- bzw. Absorbat und/oder das Ad- bzw. Absorbens chemisch verändert wird.

Ein spezifisches, physikalisches Absorptionsverfahren stellt die Gaswäsche mit tiefkaltem Methanol dar, das als Absorbens oder Waschmittel Methanol verwendet, dessen Temperatur mittels kälteerzeugender Verfahren unter die Umgebungstemperatur, bevorzugt unter 0 °C, meist bevorzugt unter - 30 °C abgekühlt wurde. Dieses Verfahren ist dem Fachmann unter der Bezeichnung Rectisol-Verfahren bekannt.

Unter einem Aufteilen eines Stoffstroms ist im Zusammenhang mit der vorliegenden Erfindung ein Aufspalten des Stroms in mindestens zwei Teilströme zu verstehen, deren stoffliche Zusammensetzung und Phasenzustand derjenigen des Ausgangsstroms entspricht. Im Gegensatz hierzu ist mit dem Auftrennen eines Stoffstroms die Aufspalten des Stroms in mindestens zwei Teilströme unter Zuhilfenahme eines Phasengleichgewichts gemeint, wobei sich die Zusammensetzung der erhaltenen Stoffströme untereinander und von derjenigen des Ausgangsstroms unterscheidet.

Für die Zwecke dieser Beschreibung ist Dampf synonym mit Wasserdampf zu verstehen, sofern es nicht im Einzelfall anders angegeben wird. Die Angabe "Wasser" bezieht sich dagegen auf Wasser im flüssigen Aggregatzustand, soweit nicht im Einzelfall anders angegeben.

Unter einem Mittel wird eine Sache verstanden, die die Erreichung eines Zieles ermöglicht oder dabei behilflich ist. Insbesondere werden unter Mitteln zum Durchführen eines bestimmten Verfahrensschrittes alle diejenigen physischen Gegenstände verstanden, die der Fachmann in Betracht ziehen würde, um diesen Verfahrensschritt durchführen zu können. Beispielsweise wird der Fachmann als Mittel zum Einleiten oder Ausleiten eines Stoffstroms alle Transport- und Fördervorrichtungen, also z. B. Rohrleitungen, Pumpen, Verdichter, Ventile sowie die entsprechenden Durchtrittsöffnungen in Behälterwänden in Betracht ziehen, die ihm aufgrund seines Fachwissens zur Durchführung dieses Verfahrensschrittes notwendig oder sinnvoll erscheinen.

Unter Fluidverbindung zwischen zwei Bereichen oder Anlagenteilen wird dabei jegliche Art von Verbindung verstanden, die es ermöglicht, dass ein Fluid, beispielsweise ein Reaktionsprodukt oder eine Kohlenwasserstofffraktion, von dem einen zu dem anderen der beiden Bereiche strömen kann, unbeachtlich etwaiger zwischengeschalteter Bereiche, Bauteile oder benötigter Fördermittel.

Alle etwaigen Druckangaben erfolgen in bar als Absolutdruckeinheiten, abgekürzt bara, oder als Überdruckeinheiten, abgekürzt barg, wenn im jeweiligen Einzelzusammenhang nichts anderes angegeben wird.

Der Erfindung liegt die Erkenntnis zugrunde, dass ausgehend von dem in der Patentschrift DE 10214003 B4 gelehrten Verfahren unter Beibehaltung der deutlichen Reduzierung der CO₂-Emissionen eine verbesserte Nutzung des abgetrennten CO₂ und weitere Vorteile erhalten werden können. Dies wird erfindungsgemäß dadurch erreicht, dass das Kohlendioxid, das aus dem zweiten Rohsynthesegasteilstrom mittels der Sorptionsvorrichtung abgetrennt wird, mindestens teilweise in das Einsatzgas für den Methanolsynthesereaktor eingeleitet wird, anstatt es zu der Partialoxidationsstufe zurückzuführen. Dies führt zu den folgenden Vorteilen:
(a) Der CO₂-Gehalt im Einsatzgas für die Methanolsynthese wird erhöht und die Stöchiometriezahl SN kann hierdurch auf den für die Methanolsynthese erwünschter Wert von etwas größer als 2, beispielsweise 2,1 eingestellt werden. Dies führt zu einer verbesserten Umsetzung im Methanolsynthesereaktor und einer höheren Effizienz der Methanolproduktion, insbesondere bei Verfahren mit Synthesegaserzeugung mittels Partialoxidation, beispielsweise mittels Erdgas-POX, bei dem der CO₂-Gehalt im Syngas an sich vergleichsweise niedrig ist.
(b) Der technische Aufwand und der Energiebedarf für die CO₂-Kompression und die Kühlung des Verdichters reduzieren sich. Je nach CO/Methanol-Verhältnis reduziert sich der Energiebedarf um ca. 15 bis 20 %.
(c) Der Sauerstoffbedarf reduziert sich.
(d) Die Steuerung der Einleitung des abgetrennten CO₂ in das im Einsatzgas für die Methanolsynthese ist weniger komplex als dessen Rückführen zum und Einleiten in den Partialoxidationsreaktor.

Indem das abgetrennte CO₂ direkt in das Einsatzgas für die Methanolsynthese eingeleitet wird, wird der CO₂-Gehalt am Eintritt in den Methanolsynthesereaktor erhöht. Insbesondere wenn leichtere Kohlenwasserstoffe wie Erdgas als Ausgangsmaterial in einem Partialoxidationsprozess verwendet werden, kann hierdurch ein vorteilhafterer CO₂-Gehalt im Einsatzgas für die Methanolsynthese erreicht werden. Die Energie für die Nachverdichtung und die Kühlung des Kompressors ist geringer als bei dem in der Patentschrift DE 10214003 B4 gelehrten Verfahren, da der CO₂-Stoffmengenstrom und der Druck am Eintritt in den Methanolsynthesereaktor niedriger sind als beim Partialoxidationsreaktor. Da das CO₂ nicht auf die Partialoxidationstemperatur aufgeheizt werden muss, wird auch weniger Sauerstoff benötigt. Dies ist auch dann der Fall, wenn etwas mehr Erdgas verwendet wird, um den etwas geringeren Rohsynthesegasstrom aus dem Partialoxidationsreaktor auszugleichen, wenn kein CO₂ und damit weniger Kohlenstoff in den Partialoxidationsreaktor geleitet wird.

### Besondere Ausgestaltungen der Erfindung

In einem zweiten Aspekt der Erfindung ist das erfindungsgemäße Verfahren dadurch gekennzeichnet, dass der Wasserstoff-Reichgasstrom mindestens teilweise in den Methanolsynthesereaktor eingeleitet wird. Hierdurch besteht eine weitere Möglichkeit zum Einstellen der gewünschten Stöchiometriezahl SN für die Methanolsynthese.

In einem dritten Aspekt der Erfindung ist das erfindungsgemäße Verfahren dadurch gekennzeichnet, dass ein solcher Anteil des Wasserstoff-Reichgasstroms in den Methanolsynthesereaktor eingeleitet wird, dass die Stöchiometriezahl SN, die sich auf die Gesamtheit aller in den Methanolsynthesereaktor eingeleiteten Stoffströme am Reaktoreingang des Methanolsynthesereaktors bezieht, zwischen 1,8 und 2,4, bevorzugt zwischen 2,0 und 2,2 liegt. Hierdurch besteht eine weitere Möglichkeit zum Einstellen der optimalen Stöchiometriezahl SN für die Methanolsynthese.

In einem vierten Aspekt der Erfindung ist das erfindungsgemäße Verfahren dadurch gekennzeichnet, dass der Wasserstoff-Reichgasstrom mindestens teilweise dem Brenner der Heizvorrichtung als zweites Heizgas zugeführt wird. Hierdurch kann die CO2-Emission des Gesamtverfahrens reduziert werden, da kohlenstoffbasierter Brennstoff partiell gegen Wasserstoff substituiert wird.

In einem fünften Aspekt der Erfindung ist das erfindungsgemäße Verfahren dadurch gekennzeichnet, dass dem Brenner der Heizvorrichtung ein Teil des Kohlenwasserstoffe enthaltenden Einsatzstroms, bevorzugt Erdgas, als drittes Heizgas zugeführt wird. Insbesondere bei Inbetriebnahme des Verfahrens bzw. der Anlage kann somit in zuverlässiger Weise Heizgas bereitgestellt werden, da brennbare Abfallströme wie beispielsweise der Methanolsynthese-Spülstrom erst nach der Inbetriebnahme des Gesamtverfahrens, insbesondere der Methanolsynthese, zur Verfügung stehen.

In einem sechsten Aspekt der Erfindung ist das erfindungsgemäße Verfahren dadurch gekennzeichnet, dass dem Brenner der Heizvorrichtung mindestens ein Teil des Methanolsynthese-Spülstroms als viertes Heizgas zugeführt wird. Auf diese Weise kann der Methanolsynthese-Spülstrom thermisch genutzt werden und enthaltene Gefahrstoffe wie beispielsweise Kohlenmonoxid werden neutralisiert. Ferner wird die Flexibilität hinsichtlich der zur Verfügung stehenden Heizgase erhöht.

In einem siebten Aspekt der Erfindung ist das erfindungsgemäße Verfahren dadurch gekennzeichnet, dass die Heizvorrichtung zur Dampferzeugung dient, wobei der erzeugte Dampf mindestens teilweise als Moderator in der Partialoxidationsstufe dient. Auf diese Weise kann die erzeugte Abwärme besser genutzt werden und der thermische Wirkungsgrad des Verfahrens bzw. der Anlage wird erhöht.

In einem achten Aspekt der Erfindung ist das erfindungsgemäße Verfahren dadurch gekennzeichnet, dass die Heizvorrichtung zur Dampferzeugung dient, wobei der erzeugte Dampf mindestens teilweise an externe Verbraucher abgegeben wird (Exportdampf). Hierdurch reduziert sich der technische und energetische Aufwand zur Dampferzeugung bei den externen Verbrauchern.

In einem neunten Aspekt der Erfindung ist das erfindungsgemäße Verfahren dadurch gekennzeichnet, dass zusätzlich ein Kohlendioxid enthaltender Gasstrom, der aus einer verfahrensexternen Quelle stammt, in den Methanolsynthesereaktor eingeleitet wird. Hierdurch steht eine Senke für das klimaschädliche Kohlendioxid zur Verfügung.

In einem zehnten Aspekt der Erfindung ist das erfindungsgemäße Verfahren dadurch gekennzeichnet, dass die Heizvorrichtung zur Vorwärmung des Kohlenwasserstoffe enthaltenden Einsatzstroms und/oder des Stroms des Sauerstoff enthaltenden Oxidationsmittels dient. Auf diese Weise kann die erzeugte Abwärme besser genutzt werden und der thermische Wirkungsgrad des Verfahrens bzw. der Anlage wird erhöht.

In einem elften Aspekt der Erfindung ist das erfindungsgemäße Verfahren dadurch gekennzeichnet, dass der Methanolsynthese-Spülstrom mittels einer Trennvorrichtung, bevorzugt einer Membrantrennvorrichtung, in einen ersten, an Wasserstoff angereicherten Spülstrom und in einen zweiten, an Wasserstoff abgereicherten und an Kohlenoxiden und Methan angereicherten Spülstrom aufgetrennt wird, wobei mindestens ein Teil des ersten, an Wasserstoff angereicherten Spülstroms dem Brenner der Heizvorrichtung als viertes Heizgas zugeführt wird und wobei mindestens ein Teil des zweiten, an Kohlenoxiden und Methan angereicherten Spülstroms zur Partialoxidationsstufe geführt wird. Auf diese Weise kann der Methanolsynthese-Spülstrom thermisch und stofflich genutzt werden und die CO₂-Emission des Verfahrens bzw. der Anlage wird reduziert.

In einem dreizehnten Aspekt der Erfindung ist die erfindungsgemäße Anlage dadurch gekennzeichnet, dass ferner Mittel umfasst werden, die es gestatten, dass der Wasserstoff-Reichgasstrom mindestens teilweise dem Brenner der Heizvorrichtung als zweites Heizgas zugeführt wird.

Die mit diesem Aspekt verbundenen technischen Effekte und Vorteile entsprechen denen, die im Zusammenhang mit dem vierten Aspekt der Erfindung erörtert wurden.

In einem vierzehnten Aspekt der Erfindung ist die erfindungsgemäße Anlage dadurch gekennzeichnet, dass ferner Mittel umfasst werden, die es gestatten, dass dem Brenner der Heizvorrichtung ein Teil des Kohlenwasserstoffe enthaltenden Einsatzstrom, bevorzugt Erdgas, als drittes Heizgas zugeführt wird.

Die mit diesem Aspekt verbundenen technischen Effekte und Vorteile entsprechen denen, die im Zusammenhang mit dem fünften Aspekt der Erfindung erörtert wurden.

In einem fünfzehnten Aspekt der Erfindung ist die erfindungsgemäße Anlage dadurch gekennzeichnet, dass ferner Mittel umfasst werden, die es gestatten, dass dem Brenner der Heizvorrichtung mindestens ein Teil des Methanolsynthese-Spülstroms als viertes Heizgas zugeführt wird.

Die mit diesem Aspekt verbundenen technischen Effekte und Vorteile entsprechen denen, die im Zusammenhang mit dem sechsten Aspekt der Erfindung erörtert wurden.

In einem sechzehnten Aspekt der Erfindung ist die erfindungsgemäße Anlage dadurch gekennzeichnet, dass ferner Mittel umfasst werden, die es gestatten, dass die Heizvorrichtung der Dampferzeugung dient, wobei der erzeugte Dampf mindestens teilweise als Moderator in der Partialoxidationsstufe verwendbar ist.

Die mit diesem Aspekt verbundenen technischen Effekte und Vorteile entsprechen denen, die im Zusammenhang mit dem siebten Aspekt der Erfindung erörtert wurden.

In einem siebzehnten Aspekt der Erfindung ist die erfindungsgemäße Anlage dadurch gekennzeichnet, dass ferner Mittel umfasst werden, die es gestatten, dass die Heizvorrichtung der Dampferzeugung dient, wobei der erzeugte Dampf mindestens teilweise an externe Verbraucher abgebbar ist (Exportdampf).

Die mit diesem Aspekt verbundenen technischen Effekte und Vorteile entsprechen denen, die im Zusammenhang mit dem achten Aspekt der Erfindung erörtert wurden.

In einem achtzehnten Aspekt der Erfindung ist die erfindungsgemäße Anlage dadurch gekennzeichnet, dass ferner Mittel umfasst werden, die es gestatten, dass zusätzlich ein Kohlendioxid enthaltender Gasstrom, der aus einer verfahrensexternen Quelle stammt, in den Methanolsynthesereaktor einleitbar ist.

Die mit diesem Aspekt verbundenen technischen Effekte und Vorteile entsprechen denen, die im Zusammenhang mit dem neunten Aspekt der Erfindung erörtert wurden.

In einem neunzehnten Aspekt der Erfindung ist die erfindungsgemäße Anlage dadurch gekennzeichnet, dass ferner Mittel umfasst werden, die es gestatten, dass die Heizvorrichtung zur Vorwärmung des Kohlenwasserstoffe enthaltenden Einsatzstroms und/oder des Stroms des Sauerstoff enthaltenden Oxidationsmittels verwendbar ist.

Die mit diesem Aspekt verbundenen technischen Effekte und Vorteile entsprechen denen, die im Zusammenhang mit dem zehnten Aspekt der Erfindung erörtert wurden.

In einem zwanzigsten Aspekt der Erfindung ist die erfindungsgemäße Anlage dadurch gekennzeichnet, dass ferner Mittel umfasst werden, die es gestatten, dass
- der Methanolsynthese-Spülstrom mittels einer Trennvorrichtung, bevorzugt einer Membrantrennvorrichtung, in einen ersten, an Wasserstoff angereicherten Spülstrom und in einen zweiten, an Wasserstoff abgereicherten und an Kohlenoxiden und Methan angereicherten Spülstrom auftrennbar ist,
- mindestens ein Teil des ersten, an Wasserstoff angereicherten Spülstroms dem Brenner der Heizvorrichtung als viertes Heizgas zuführbar ist,
- mindestens ein Teil des zweiten, an Kohlenoxiden und Methan angereicherten Spülstroms zur Partialoxidationsstufe zuführbar ist.

Die mit diesem Aspekt verbundenen technischen Effekte und Vorteile entsprechen denen, die im Zusammenhang mit dem elften Aspekt der Erfindung erörtert wurden.

### Ausführungs- und Zahlenbeispiele

Weiterbildungen, Vorteile und Anwendungsmöglichkeiten der Erfindung ergeben sich auch aus der nachfolgenden Beschreibung von Ausführungs- und Zahlenbeispielen und den Zeichnungen. Dabei bilden alle beschriebenen und/oder bildlich dargestellten Merkmale für sich oder in beliebiger Kombination die Erfindung, unabhängig von ihrer Zusammenfassung in den Ansprüchen oder deren Rückbeziehung.

Es zeigt die einzige Figur:

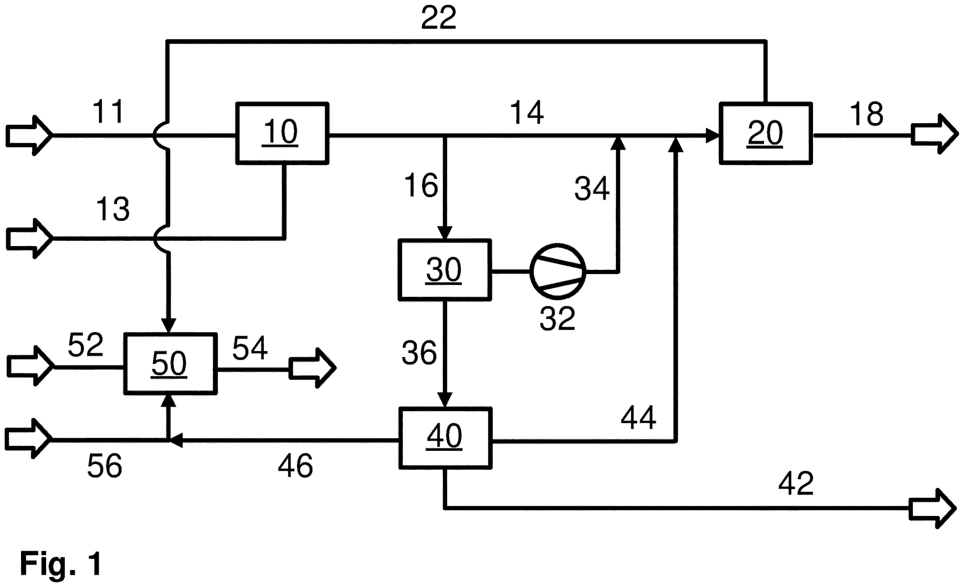

- Fig. 1: eine schematische Darstellung des Verfahrens bzw. der Anlage gemäß einer Ausführungsform der Erfindung.

In der in Fig. 1 dargestellten Ausgestaltung eines Verfahrens bzw. einer Anlage gemäß der Erfindung wird über Leitung 11 ein Kohlenwasserstoffe enthaltender Einsatzstrom, beispielsweise Erdgas, in einem bevorzugten Beispiel Erdgas mit einem Methangehalt von mindestens 80 Vol.-%, einer nichtkatalytischen Partialoxidationsstufe (POX) 10 zugeführt. Der für die Partialoxidation als Oxidationsmittel benötigte Sauerstoff wird über Leitung 13 der Partialoxidationsstufe zugeführt. Die nichtkatalytische Partialoxidationsstufe wird unter an sich bekannten Partialoxidationsbedingungen betrieben. Als zusätzliches Betriebsmedium wird der POX-Stufe optional Wasserdampf und/oder Kohlendioxid als Moderator zugeführt.

In einem weiteren, bildlich nicht gezeigten Beispiel kann die Partialoxidationsstufe als Autothermreformer (ATR) ausgestaltet werden, der in einem Beispiel bei einem Druck von 60 bara betrieben wird. Als zusätzliches Betriebsmedium wird dem ATR optional Wasserdampf und/oder Kohlendioxid als Moderator zugeführt.

In der Partialoxidationsstufe 10 erfolgt ein mindestens teilweises Umsetzen des Kohlenwasserstoffe enthaltenden Einsatzstroms unter Synthesegaserzeugungsbedingungen zu einem Wasserstoff (H₂), Kohlenmonoxid (CO) und im Sinne der Methanolsynthese inerter Komponenten wie Methan (CH₄) enthaltenden Rohsynthesegasstrom, der aus der Partialoxidationsstufe ausgeleitet und in einen ersten Rohsynthesegasteilstrom (Leitung 14) und in einen zweiten Rohsynthesegasteilstrom (Leitung 16) aufgeteilt wird.

Über Leitung 14 wird der erste Rohsynthesegasteilstrom einem Methanolsynthesereaktor 20 zugeführt, in dem ein mindestens teilweises Umsetzen des ersten Rohsynthesegasteilstroms unter Methanolsynthesebedingungen folgt. Das dabei erhaltene Rohmethanolprodukt wird über Leitung 18 aus dem Methanolsynthesereaktor 20 ausgeleitet und der Weiterverarbeitung, Aufbereitung, Lagerung oder einem Verbraucher zugeführt.

Für die Zwecke der vorliegenden Beschreibung ist der Begriff "Methanolsynthesereaktor" und das Bezugszeichen 20 so zu verstehen, dass hiervon sowohl der oder die katalytischen Reaktoren zur Methanolsynthese, aber auch weitere übliche und dem Fachmann geläufige, bildlich nicht gezeigte Bestandteile einer Methanolsyntheseeinheit umfasst werden:
- Leitungen und mindestens ein Verdichter zum Aufbau eines Kreislaufs für nicht umgesetztes Synthesegas,
- Kühler zum Abkühlen des Reaktorproduktstroms des Methanolsynthesereaktors,
- eine Phasentrennvorrichtung zum Auftrennen des abgekühlten Reaktorproduktstroms des Methanolsynthesereaktors in einen ersten Flüssigproduktstrom und einen ersten Restgasstrom, der nicht umgesetzte Synthesegasbestandteile und Inertkomponenten enthält,
- eine Vorrichtung zum Aufteilen des ersten Restgasstroms in einen Methanolsynthese-Spülstrom und in einen Recyclestrom, der zum Methanolsynthesereaktor zurückgeführt wird.

Über Leitung 22 wird der Methanolsynthese-Spülstrom aus dem Methanolsynthesereaktor ausgeleitet.

Über Leitung 16 wird der zweite Rohsynthesegasteilstrom in eine Sorptionsvorrichtung 30 zur Entfernung von Kohlendioxid eingeleitet. In einem Beispiel arbeitet die Sorptionsvorrichtung nach einem physikalischen Sorptionsverfahren, das tiefkaltes Methanol als Absorbens bzw. Waschmedium verwendet (Rectisol-Verfahren). Einzelheiten dieses Verfahrens sind dem Fachmann bekannt. Hierdurch ergeben sich weitere synergistische Vorteile, da in einem Beispiel ein Teil des in dem Methanolsynthesereaktor 20 erzeugten Methanols als Waschmedium verwendet werden kann. In einem Beispiel können Teile der Vorrichtungen zur Aufbereitung des über Leitung 18 aus dem Methanolsynthesereaktor 20 ausgeleiteten Rohmethanolprodukts auch zur Regenerierung des mit Kohlendioxid beladenen Waschmediums verwendet werden. In einem Beispiel wird Abwärme aus den Vorrichtungen zur Aufbereitung des Rohmethanolprodukts zum Erhitzen oder Vorwärmen des mit Kohlendioxid beladenen Waschmediums verwendet, beispielsweise zum Zwecke der Regenerierung.

Für die Zwecke der vorliegenden Beschreibung ist der Begriff "Sorptionsvorrichtung" und das Bezugszeichen 30 so zu verstehen, dass hiervon sowohl die eigentliche Entfernung bzw. Abtrennung von Kohlendioxid, aber auch die Regenerierung des verwendeten Sorptionsmittels und die Gewinnung eines an Kohlendioxid angereicherten Gasstroms umfasst werden. Der an Kohlendioxid angereicherte Gasstrom wird in einem Verdichter 32, der im Leitungsweg der Leitung 34 angeordnet ist, auf Methanolsynthesedruck verdichtet und erfindungsgemäß über Leitung 34 dem Methanolsynthesereaktor 20 zugeführt. In einem Beispiel mündet Leitung 34 in Leitung 14 ein, mittels der der erste Rohsynthesegasteilstrom in den Methanolsynthesereaktor eingeleitet wird. In einem Beispiel mündet Leitung 34 direkt in den Methanolsynthesereaktor ein.

Über Leitung 36 wird ein an Kohlendioxid abgereicherter Synthesegasstrom aus der Sorptionsvorrichtung 30 ausgeleitet, zu einer kryogenen Gaszerlegungsstufe 40 geführt und in diese eingeleitet. In der kryogenen Gaszerlegungsstufe erfolgt das Auftrennen des an Kohlendioxid abgereicherten Synthesegasstroms in folgende Teilströme:
(1) Einen Kohlenmonoxid-Reichgasstrom. Dieser wird über Leitung 42 als Kohlenmonoxid-Produktstrom aus dem Verfahren ausgeleitet.
(2) Einen Wasserstoff-Reichgasstrom. Dieser wird über Leitung 44 dem Methanolsynthesereaktor 20 zugeführt und dort zum Einstellen der gewünschten Stöchiometriezahl für die Methanolsynthese verwendet. In einem Beispiel liegt die somit eingestellte Stöchiometriezahl zwischen 1,8 und 2,4, bevorzugt zwischen 2,0 und 2,2. In einem Beispiel beträgt die somit eingestellte Stöchiometriezahl 2,1.

In einem Beispiel (bildlich nicht gezeigt) wird der Wasserstoff-Reichgasstrom mindestens teilweise einem Brenner einer Heizvorrichtung 50 als Heizgas zugeführt. In einem Beispiel (bildlich nicht gezeigt) wird der gesamte Wasserstoff-Reichgasstrom dem Brenner der Heizvorrichtung 50 als Heizgas zugeführt, In einem Beispiel (bildlich nicht gezeigt) wird derjenige Anteil des Wasserstoff-Reichgasstroms dem Brenner der Heizvorrichtung 50 als Heizgas zugeführt, der zum Einstellen der gewünschten Stöchiometriezahl für die Methanolsynthese nicht benötigt wird.

In einem Beispiel mündet Leitung 44 in Leitung 14 ein, mittels der der erste Rohsynthesegasteilstrom in den Methanolsynthesereaktor eingeleitet wird. In einem Beispiel mündet Leitung 44 direkt in den Methanolsynthesereaktor ein.

(3) Einen Inertkomponenten, Methan, Wasserstoff und Kohlenmonoxid enthaltenden Abgasstrom. Dieser wird mindestens teilweise über Leitung 46 einem Brenner einer Heizvorrichtung 50 als Heizgas zugeführt. Dem Brenner der Heizvorrichtung 50 wird ferner über Leitung 56 ein Kohlenwasserstoffe enthaltender Einsatzstrom, beispielsweise Erdgas, als Brenngas zugeführt. In einem Beispiel wird dem Brenner der Heizvorrichtung 50 ferner über Leitung 22 mindestens ein Teil des Methanolsynthese-Spülstroms aus dem Methanolsynthesereaktor als Brenngas zugeführt.

Die Heizvorrichtung 50 wird in einem Beispiel zur Dampferzeugung verwendet. Hierzu wird über Leitung 52 Kesselspeisewasser in die Heizvorrichtung 50 eingeleitet und in dieser verdampft. Der erzeugte Dampf wird über Leitung 54 aus der Heizvorrichtung 50 ausgeleitet. In einem Beispiel wird ein Teil des erzeugten Dampfs in der Partialoxidationsstufe 10 als Moderator verwendet. In einem Beispiel wird mindestens ein Teil des erzeugten Dampfs an externe Verbraucher abgegeben (Exportdampf).

Weitere Vorteile und eine noch flexiblere Verfahrensgestaltung ergeben sich aus den folgenden Beispielen, die mit dem Grundverfahren gemäß der Erfindung kombinierbar sind:
In einem Beispiel wird zusätzlich Kohlendioxid aus einer verfahrensexternen CO₂-Quelle in die Partialoxidationsstufe eingeleitet. Dies ist insbesondere dann vorteilhaft, wenn der verfahrensexterne CO₂-Strom mit erhöhtem Druck zur Verfügung steht, so dass der Kompressionsaufwand vor Einleiten in die Partialoxidationsstufe minimiert wird oder sogar ganz entfällt.

In einem Beispiel wird zusätzlich Kohlendioxid aus einer verfahrensexternen CO₂-Quelle in den Methanolsynthesereaktor eingeleitet.

In einem Beispiel wird Wasserstoff aus einer verfahrensexternen Wasserstoffquelle verwendet, um die gewünschte Stöchiometriezahl für die Methanolsynthese einzustellen.

### Zahlenbeispiel

Die nachfolgende Tabelle zeigt einen Vergleich berechneter Kenndaten der Erfindung mit einem Verfahrensschema gemäß Stand der Technik (DE 10214003 B4) für eine vorgegebene Produktionsmenge von CO und Methanol.

**Tabelle: Vergleich berechneter Kenndaten der Erfindung mit einem Verfahrensschema gemäß Stand der Technik (DE 10214003 B4) für eine vorgegebene Produktionsmenge von CO und Methanol.**

| | | **Vergleichsbeispiel** | **Erfindung** |
|---|---|---|---|
| CO-Produktion | kg/h | 14560 | 14558 |
| MeOH-Produktion | kg/h | 34296 | 34308 |
| | | | |
| Erdgas-Zufuhr | kg/h | 27914 | 27999 |
| Sauerstoff-Zufuhr | kg/h | 35860 | 35621 |
| kgSyngas/kgErdgas | | 2,071 | 2,015 |
| Synthesegas zu CO | kg/h | 25949 | 25828 |
| Synthesegas zu MeOH | kg/h | 31861 | 30585 |
| | | | |
| CO2-Rückführung | kg/h | 2181 | 1938 |
| Verdichterleistung | kW | 160 | 130 |
| Verdichter Kühlleistung | kW | 140 | 117 |
| | | | |
| Feedstrom MeOH | kg/h | 36856 | 37646 |
| Stöchiometriezahl | | 2,085 | 2,086 |
| CO2 zu MeOH Synthese | Mol% | 1,76% | 2,74% |
| CO zu MeOH Synthese | Mol% | 29,74% | 28,43% |
| H2 zu MeOH Synthese | Mol% | 67,43% | 67,75% |
| CO/CO2 zu MeOH Synth. | | 16,9 | 10,4 |
| | | | |
| Überhitzter Dampf 40 bara | kg/h | 127431 | 124781 |

Mit der Erfindung werden folgende Vorteile gegenüber dem Stand der Technik erhalten:
- Bereitstellung eines Gases mit höherem CO₂-Gehalt, das für die Methanolsynthese besser geeignet ist, was zu einer höheren Effizienz der Methanolsynthese führt.
- Weniger CO₂ muss auf einen niedrigeren Druck komprimiert werden. Die Einsparung hinsichtlich der Verdichterleistung und der Kühlleistung beträgt ca. 15 bis 20 %.
- Der Sauerstoffbedarf sinkt um ca. 0,5 bis 0,8 %.
- Eine um ca. 2 % geringere überschüssige Dampferzeugung kann von Vorteil sein, wenn keine externe Nutzung von Dampf und somit kein Dampfexport erwünscht ist.

### Bezugszeichenliste

- [10]: Partialoxidationsstufe
- [11]: Leitung
- [13]: Leitung
- [14]: Leitung
- [16]: Leitung
- [18]: Leitung
- [20]: Methanolsynthesereaktor
- [22]: Leitung
- [30]: Sorptionsvorrichtung
- [32]: Verdichter
- [34]: Leitung
- [36]: Leitung
- [40]: kryogene Gaszerlegungsstufe
- [42]: Leitung
- [44]: Leitung
- [46]: Leitung
- [50]: Heizvorrichtung
- [52]: Leitung
- [54]: Leitung
- [56]: Leitung

## Patentansprüche

1. Verfahren zum Herstellen von Methanol und reinem Kohlenmonoxid aus einem Kohlenwasserstoffe enthaltenden Einsatzstrom, bevorzugt Erdgas, umfassend folgende Schritte:
(a) Bereitstellen des Kohlenwasserstoffe enthaltenden Einsatzstroms,
(b) Zuführen des Kohlenwasserstoffe enthaltenden Einsatzstroms zu einer Partialoxidationsstufe (POX),
(c) mindestens teilweises Umsetzen des Kohlenwasserstoffe enthaltenden Einsatzstroms in der Partialoxidationsstufe mit einem Strom eines Sauerstoff enthaltenden Oxidationsmittels unter Partialoxidationsbedingungen und unter optionaler Anwesenheit eines Moderatorstroms, bevorzugt umfassend Dampf und/oder Kohlendioxid, zu einem Wasserstoff (H₂), Kohlenmonoxid (CO), Kohlendioxid (CO₂) und Methan (CH₄) enthaltenden Rohsynthesegasstrom,
(d) Ausleiten des Rohsynthesegasstroms aus der Synthesegaserzeugungsanlage und Aufteilen des Rohsynthesegasstroms in einen ersten Rohsynthesegasteilstrom und in einen zweiten Rohsynthesegasteilstrom,
(e) Einleiten mindestens eines Teils des ersten Rohsynthesegasteilstroms in einen Methanolsynthesereaktor, mindestens teilweises Umsetzen des ersten Rohsynthesegasteilstroms in dem Methanolsynthesereaktor unter Methanolsynthesebedingungen,
(f) Ausleiten eines Methanol enthaltenden ersten Reaktorproduktstroms aus dem Methanolsynthesereaktor, Abkühlen des ersten Reaktorproduktstroms unter seinen Taupunkt und Auftrennen des abgekühlten ersten Reaktorproduktstroms in einer Phasentrennvorrichtung in einen ersten Flüssigproduktstrom und einen ersten Restgasstrom, der nicht umgesetzte Synthesegasbestandteile und Inertkomponenten enthält, Ausleiten des ersten Flüssigproduktstroms aus dem Verfahren als Rohmethanolproduktstrom,
(g) Aufteilen des ersten Restgasstroms in einen Methanolsynthese-Spülstrom und in einen Recyclestrom, der zum Methanolsynthesereaktor zurückgeführt wird,
(h) Einleiten mindestens eines Teils des zweiten Rohsynthesegasteilstroms in eine Sorptionsvorrichtung zur Entfernung von Kohlendioxid mittels eines physikalischen oder chemischen Sorptionsverfahrens, Ausleiten eines an Kohlendioxid abgereicherten Synthesegasstroms und eines an Kohlendioxid angereicherten Gasstroms aus der Sorptionsvorrichtung,
(j) Einleiten mindestens eines Teils des Kohlendioxid abgereicherten Synthesegasstroms in eine kryogene Gaszerlegungsstufe, Auftrennen des an Kohlendioxid abgereicherten Synthesegasstroms in der kryogenen Gaszerlegungsstufe in folgende Teilströme:
(j1) einen Kohlenmonoxid-Reichgasstrom, der als Kohlenmonoxid-Produktstrom aus dem Verfahren ausgeleitet wird,
(j2) einen Wasserstoff-Reichgasstrom,
(j3) einen Inertkomponenten, Methan, Wasserstoff und Kohlenmonoxid enthaltenden Abgasstrom, der mindestens teilweise einem Brenner einer Heizvorrichtung als erstes Heizgas zugeführt wird,
**dadurch gekennzeichnet, dass** der an Kohlendioxid angereicherte Gasstrom mindestens teilweise in den Methanolsynthesereaktor eingeleitet wird.

2. Verfahren nach Anspruch 1, **dadurch gekennzeichnet, dass** der Wasserstoff-Reichgasstrom mindestens teilweise in den Methanolsynthesereaktor eingeleitet wird.

3. Verfahren nach Anspruch 2, **dadurch gekennzeichnet, dass** ein solcher Anteil des Wasserstoff-Reichgasstroms in den Methanolsynthesereaktor eingeleitet wird, dass die Stöchiometriezahl SN, die sich auf die Gesamtheit aller in den Methanolsynthesereaktor eingeleiteten Stoffströme am Reaktoreingang des Methanolsynthesereaktors bezieht, zwischen 1,8 und 2,4, bevorzugt zwischen 2,0 und 2,2 liegt.

4. Verfahren nach Anspruch 1 oder 2 oder 3, **dadurch gekennzeichnet, dass** der Wasserstoff-Reichgasstrom mindestens teilweise dem Brenner der Heizvorrichtung als zweites Heizgas zugeführt wird.

5. Verfahren nach einem der vorigen Ansprüche, **dadurch gekennzeichnet, dass** dem Brenner der Heizvorrichtung ein Teil des Kohlenwasserstoffe enthaltenden Einsatzstrom, bevorzugt Erdgas, als drittes Heizgas zugeführt wird.

6. Verfahren nach einem der vorigen Ansprüche, **dadurch gekennzeichnet, dass** dem Brenner der Heizvorrichtung mindestens ein Teil des Methanolsynthese-Spülstroms als viertes Heizgas zugeführt wird.

7. Verfahren nach einem der vorigen Ansprüche, **dadurch gekennzeichnet, dass** die Heizvorrichtung zur Dampferzeugung dient, wobei der erzeugte Dampf mindestens teilweise als Moderator in der Partialoxidationsstufe dient.

8. Verfahren nach einem der vorigen Ansprüche, **dadurch gekennzeichnet, dass** die Heizvorrichtung zur Dampferzeugung dient, wobei der erzeugte Dampf mindestens teilweise an externe Verbraucher abgegeben wird (Exportdampf).

9. Verfahren nach einem der vorigen Ansprüche, **dadurch gekennzeichnet, dass** zusätzlich ein Kohlendioxid enthaltender Gasstrom, der aus einer verfahrensexternen Quelle stammt, in den Methanolsynthesereaktor eingeleitet wird.

10. Verfahren nach einem der vorigen Ansprüche, **dadurch gekennzeichnet, dass** die Heizvorrichtung zur Vorwärmung des Kohlenwasserstoffe enthaltenden Einsatzstroms und/oder des Stroms des Sauerstoff enthaltenden Oxidationsmittels dient.

11. Verfahren nach einem der vorigen Ansprüche, **dadurch gekennzeichnet, dass** der Methanolsynthese-Spülstrom mittels einer Trennvorrichtung, bevorzugt einer Membrantrennvorrichtung, in einen ersten, an Wasserstoff angereicherten Spülstrom und in einen zweiten, an Wasserstoff abgereicherten und an Kohlenoxiden und Methan angereicherten Spülstrom aufgetrennt wird, wobei mindestens ein Teil des ersten, an Wasserstoff angereicherten Spülstroms dem Brenner der Heizvorrichtung als viertes Heizgas zugeführt wird und wobei mindestens ein Teil des zweiten, an Kohlenoxiden und Methan angereicherten Spülstroms zur Partialoxidationsstufe geführt wird.

12. Anlage zum Herstellen von Methanol und reinem Kohlenmonoxid aus einem Kohlenwasserstoffe enthaltenden Einsatzstrom, bevorzugt Erdgas, umfassend folgende, miteinander in Fluidverbindung stehende Bestandteile:
(a) Mittel zum Bereitstellen des Kohlenwasserstoffe enthaltenden Einsatzstroms,
(b) Mittel zum Zuführen des Kohlenwasserstoffe enthaltenden Einsatzstroms zu einer Partialoxidationsstufe (POX),
(c) eine Partialoxidationsstufe, Mittel zum Zuführen eines Stroms eines Sauerstoff enthaltenden Oxidationsmittels zu der Partialoxidationsstufe, optional Mittel zum Zuführen eines Stroms eines Moderatorstroms zu der Partialoxidationsstufe, Mittel zum Ausleiten eines Wasserstoff (H₂), Kohlenmonoxid (CO), Kohlendioxid (CO₂) sowie Methan (CH₄) enthaltenden Rohsynthesegasstroms aus der Partialoxidationsstufe,
(d) Mittel zum Aufteilen des Rohsynthesegasstroms in einen ersten Rohsynthesegasteilstrom und in einen zweiten Rohsynthesegasteilstrom,
(e) einen Methanolsynthesereaktor, Mittel zum Einleiten mindestens eines Teils des ersten Rohsynthesegasteilstroms in den Methanolsynthesereaktor,
(f) Mittel zum Ausleiten eines Methanol enthaltenden ersten Reaktorproduktstroms aus dem Methanolsynthesereaktor, Mittel zum Abkühlen des ersten Reaktorproduktstroms unter seinen Taupunkt, eine Phasentrennvorrichtung zum Auftrennen des abgekühlten ersten Reaktorproduktstroms in einen ersten Flüssigproduktstrom und einen ersten Restgasstrom, der nicht umgesetzte Synthesegasbestandteile und Inertkomponenten enthält, Mittel zum Ausleiten des ersten Flüssigproduktstroms aus dem Verfahren als Rohmethanolproduktstrom,
(g) Mittel zum Aufteilen des ersten Restgasstroms in einen Methanolsynthese-Spülstrom und in einen Recyclestrom, der zum Methanolsynthesereaktor zurückgeführt wird,
(h) eine Sorptionsvorrichtung zur Entfernung von Kohlendioxid mittels eines physikalischen oder chemischen Sorptionsverfahrens, eine Heizvorrichtung mit mindestens einem Brenner, Mittel zum Einleiten mindestens eines Teils des zweiten Rohsynthesegasteilstroms in die Sorptionsvorrichtung, Mittel zum Ausleiten eines an Kohlendioxid abgereicherten Synthesegasstroms und eines an Kohlendioxid angereicherten Gasstroms aus der Sorptionsvorrichtung,
(j) eine kryogene Gaszerlegungsstufe, geeignet zum Auftrennen des an Kohlendioxid abgereicherten Synthesegasstroms in der kryogenen Gaszerlegungsstufe in folgende Teilströme:
(j1) einen Kohlenmonoxid-Reichgasstrom, der als Kohlenmonoxid-Produktstrom aus dem Verfahren ausgleitbar ist,
(j2) einen Wasserstoff-Reichgasstrom,
(j3) einen Methan, Wasserstoff und Kohlenmonoxid enthaltenden Abgasstrom, der mindestens teilweise dem mindestens einen Brenner der Heizvorrichtung als erstes Heizgas einleitbar ist, Mittel zum Einleiten mindestens eines Teils des Kohlendioxid abgereicherten Synthesegasstroms in die kryogene Gaszerlegungsstufe,
**dadurch gekennzeichnet, dass** ferner Mittel umfasst werden, die es gestatten, dass der an Kohlendioxid angereicherte Gasstrom mindestens teilweise in den Methanolsynthesereaktor eingeleitet wird.

13. Anlage nach Anspruch 12, **dadurch gekennzeichnet, dass** ferner Mittel umfasst werden, die es gestatten, dass der Wasserstoff-Reichgasstrom mindestens teilweise dem Brenner der Heizvorrichtung als zweites Heizgas zugeführt wird.

14. Anlage nach einem der vorigen Ansprüche, **dadurch gekennzeichnet, dass** ferner Mittel umfasst werden, die es gestatten, dass dem Brenner der Heizvorrichtung ein Teil des Kohlenwasserstoffe enthaltenden Einsatzstrom, bevorzugt Erdgas, als drittes Heizgas zugeführt wird.

15. Anlage nach einem der vorigen Ansprüche, **dadurch gekennzeichnet, dass** ferner Mittel umfasst werden, die es gestatten, dass dem Brenner der Heizvorrichtung mindestens ein Teil des Methanolsynthese-Spülstroms als viertes Heizgas zugeführt wird.

16. Anlage nach einem der vorigen Ansprüche, **dadurch gekennzeichnet, dass** ferner Mittel umfasst werden, die es gestatten, dass die Heizvorrichtung der Dampferzeugung dient, wobei der erzeugte Dampf mindestens teilweise als Moderator in der Partialoxidationsstufe verwendbar ist.

17. Anlage nach einem der vorigen Ansprüche, **dadurch gekennzeichnet, dass** ferner Mittel umfasst werden, die es gestatten, dass die Heizvorrichtung der Dampferzeugung dient, wobei der erzeugte Dampf mindestens teilweise an externe Verbraucher abgebbar ist (Exportdampf).

18. Anlage nach einem der vorigen Ansprüche, **dadurch gekennzeichnet, dass** ferner Mittel umfasst werden, die es gestatten, dass zusätzlich ein Kohlendioxid enthaltender Gasstrom, der aus einer verfahrensexternen Quelle stammt, in den Methanolsynthesereaktor einleitbar ist.

19. Anlage nach einem der vorigen Ansprüche, **dadurch gekennzeichnet, dass** ferner Mittel umfasst werden, die es gestatten, dass die Heizvorrichtung zur Vorwärmung des Kohlenwasserstoffe enthaltenden Einsatzstroms und/oder des Stroms des Sauerstoff enthaltenden Oxidationsmittels verwendbar ist.

20. Anlage nach einem der vorigen Ansprüche, **dadurch gekennzeichnet, dass** ferner Mittel umfasst werden, die es gestatten, dass
- der Methanolsynthese-Spülstrom mittels einer Trennvorrichtung, bevorzugt einer Membrantrennvorrichtung, in einen ersten, an Wasserstoff angereicherten Spülstrom und in einen zweiten, an Wasserstoff abgereicherten und an Kohlenoxiden und Methan angereicherten Spülstrom auftrennbar ist,
- mindestens ein Teil des ersten, an Wasserstoff angereicherten Spülstroms dem Brenner der Heizvorrichtung als viertes Heizgas zuführbar ist,
- mindestens ein Teil des zweiten, an Kohlenoxiden und Methan angereicherten Spülstroms zur Partialoxidationsstufe zuführbar ist.

## Claims

1. Process for producing methanol and pure carbon monoxide from an input stream containing hydrocarbons, preferably natural gas, comprising the steps of:
(a) providing the input stream containing hydrocarbons,
(b) supplying the input stream containing hydrocarbons to a partial oxidation stage (POX),
(c) at least partial reaction of the input stream containing hydrocarbons in the partial oxidation stage with a stream of an oxygen-containing oxidant under partial oxidation conditions and in the optional presence of a moderator stream, preferably comprising steam and/or carbon dioxide, to afford a raw synthesis gas stream containing hydrogen (H₂), carbon monoxide (CO), carbon dioxide (CO₂) and methane (CH₄),
(d) discharging the raw synthesis gas stream from the synthesis gas production plant and dividing the raw synthesis gas stream into a first raw synthesis gas substream and into a second raw synthesis gas substream,
(e) introducing at least a portion of the first raw synthesis gas substream into a methanol synthesis reactor, at least partially converting the first raw synthesis gas substream in the methanol synthesis reactor under methanol synthesis conditions,
(f) discharging a methanol-containing first reactor product stream from the methanol synthesis reactor, cooling the first reactor product stream to below its dew point and separating the cooled first reactor product stream in a phase separation apparatus into a first liquid product stream and a first residual gas stream containing unconverted synthesis gas constituents and inert components, discharging the first liquid product stream from the process as a raw methanol product stream,
(g) dividing the first residual gas stream into a methanol synthesis purge stream and into a recycle stream which is recycled to the methanol synthesis reactor,
(h) introducing at least a portion of the second raw synthesis gas substream into a sorption apparatus for removal of carbon dioxide using a physical or chemical sorption process, discharging a carbon dioxide-depleted synthesis gas stream and a carbon dioxide-enriched gas stream from the sorption apparatus,
(j) introducing at least a portion of the carbon dioxide-depleted synthesis gas stream into a cryogenic gas fractionation stage, separating the carbon dioxide-depleted synthesis gas stream in the cryogenic gas fractionation stage into the following substreams:
(j1) a carbon monoxide-rich gas stream which is discharged from the process as a carbon monoxide product stream,
(j2) a hydrogen-rich gas stream,
(j3) an offgas stream containing an inert component, methane, hydrogen and carbon monoxide which is at least partially supplied to a burner of a heating apparatus as a first heating gas,
**characterized in that** the carbon dioxide-enriched gas stream is at least partially introduced into the methanol synthesis reactor.

2. Process according to Claim 1, **characterized in that** the hydrogen-rich gas stream is at least partially introduced into the methanol synthesis reactor.

3. Process according to Claim 2, **characterized in that** a proportion of the hydrogen-rich gas stream such that the stoichiometry number SN which relates to the entirety of all material streams introduced into the methanol synthesis reactor at the reactor inlet of the methanol synthesis reactor is between 1.8 and 2.4, preferably between 2.0 and 2.2, is introduced into the methanol synthesis reactor.

4. Process according to Claim 1 or 2 or 3, **characterized in that** the hydrogen-rich gas stream is at least partially supplied to the burner of the heating apparatus as a second heating gas.

5. Process according to any of the preceding claims, **characterized in that** a portion of the input stream containing hydrocarbons, preferably natural gas, is supplied to the burner of the heating apparatus as a third heating gas.

6. Process according to any of the preceding claims, **characterized in that** at least a portion of the methanol synthesis purge stream is supplied to the burner of the heating apparatus as a fourth heating gas.

7. Process according to any of the preceding claims, **characterized in that** the heating apparatus is used for steam production, wherein the steam produced is at least partially used as a moderator in the partial oxidation stage.

8. Process according to any of the preceding claims, **characterized in that** the heating apparatus is used for steam production, wherein the steam produced is at least partially provided to external consumers (export steam).

9. Process according to any of the preceding claims, **characterized in that** a carbon dioxide-containing gas stream deriving from a process-external source is additionally introduced into the methanol synthesis reactor.

10. Process according to any of the preceding claims, **characterized in that** the heating apparatus is used for preheating the input stream containing hydrocarbons and/or the stream of the oxygen-containing oxidant.

11. Process according to any of the preceding claims, **characterized in that** the methanol synthesis purge stream is separated using a separation apparatus, preferably a membrane separation apparatus, into a first purge stream enriched in hydrogen and into a second purge stream depleted in hydrogen and enriched in carbon oxides and methane, wherein at least a portion of the first purge stream enriched in hydrogen is supplied to the burner of the heating apparatus as a fourth heating gas and wherein at least a portion of the second purge stream enriched in carbon oxides and methane is passed to the partial oxidation stage.

12. Plant for producing methanol and pure carbon monoxide from an input stream containing hydrocarbons, preferably natural gas, comprising the following constituents in fluid connection with one another:
(a) means for providing the input stream containing hydrocarbons,
(b) means for supplying the input stream containing hydrocarbons to a partial oxidation stage (POX),
(c) a partial oxidation stage, means for supplying a stream of an oxygen-containing oxidant to the partial oxidation stage, optionally means for supplying a stream of a moderator stream to the partial oxidation stage, means for discharging a raw synthesis gas stream containing hydrogen (H₂), carbon monoxide (CO), carbon dioxide (CO₂) and methane (CH₄) from the partial oxidation stage,
(d) means for dividing the raw synthesis gas stream into a first raw synthesis gas substream and into a second raw synthesis gas substream,
(e) a methanol synthesis reactor, means for introducing at least a portion of the first raw synthesis gas substream into the methanol synthesis reactor,
(f) means for discharging a methanol-containing first reactor product stream from the methanol synthesis reactor, means for cooling the first reactor product stream to below its dew point, a phase separation apparatus for separating the cooled first reactor product stream into a first liquid product stream and a first residual gas stream containing unconverted synthesis gas constituents and inert components, means for discharging the first liquid product stream from the process as a raw methanol product stream,
(g) means for dividing the first residual gas stream into a methanol synthesis purge stream and into a recycle stream which is recycled to the methanol synthesis reactor,
(h) a sorption apparatus for removal of carbon dioxide using a physical or chemical sorption process, a heating apparatus having at least one burner, means for introducing at least a portion of the second raw synthesis gas substream into the sorption apparatus, means for discharging a carbon dioxide-depleted synthesis gas stream and a carbon dioxide-enriched gas stream from the sorption apparatus,
(j) a cryogenic gas fractionation stage suitable for separation of the carbon dioxide-depleted synthesis gas stream in the cryogenic gas fractionation stage into the following substreams:
(j1) a carbon monoxide-rich gas stream which is dischargeable from the process as a carbon monoxide product stream,
(j2) a hydrogen-rich gas stream,
(j3) an offgas stream containing methane, hydrogen and carbon monoxide which is at least partially introducible to the at least one burner of the heating apparatus as a first heating gas, means for introducing at least a portion of the carbon dioxide-depleted synthesis gas stream into the cryogenic gas fractionation stage,
**characterized in that** it further comprises means which allow the carbon dioxide-enriched gas stream to be at least partially introduced into the methanol synthesis reactor.

13. Plant according to Claim 12, **characterized in that** it further comprises means which allow the hydrogen-rich gas stream to be at least partially supplied to the burner of the heating apparatus as a second heating gas.

14. Plant according to any of the preceding claims, **characterized in that** it further comprises means which allow a portion of the input stream containing hydrocarbons, preferably natural gas, to be supplied to the burner of the heating apparatus as a third heating gas.

15. Plant according to any of the preceding claims, **characterized in that** it further comprises means which allow at least a portion of the methanol synthesis purge stream to be supplied to the burner of the heating apparatus as a fourth heating gas.

16. Plant according to any of the preceding claims, **characterized in that** it further comprises means which allow the heating apparatus to be used for steam production, wherein the steam produced is at least partially usable as a moderator in the partial oxidation stage.

17. Plant according to any of the preceding claims, **characterized in that** it further comprises means which allow the heating apparatus to be used for steam production, wherein the steam produced is at least partially providable to external consumers (export steam).

18. Plant according to any of the preceding claims, **characterized in that** it further comprises means which allow a carbon dioxide-containing gas stream deriving from a process-external source to be additionally introducible into the methanol synthesis reactor.

19. Plant according to any of the preceding claims, **characterized in that** it further comprises means which allow the heating apparatus to be usable for preheating the input stream containing hydrocarbons and/or the stream of the oxygen-containing oxidant.

20. Plant according to any of the preceding claims, **characterized in that** it further comprises means which allow
- the methanol synthesis purge stream to be separable using a separation apparatus, preferably a membrane separation apparatus, into a first purge stream enriched in hydrogen and into a second purge stream depleted in hydrogen and enriched in carbon oxides and methane,
- at least a portion of the first purge stream enriched in hydrogen to be suppliable to the burner of the heating apparatus as a fourth heating gas,
- at least a portion of the second purge stream enriched in carbon oxides and methane to be suppliable to the partial oxidation stage.

## Revendications

1. Procédé pour la production de méthanol et monoxyde de carbone pur à partir d'un courant de départ contenant des hydrocarbures, de préférence de gaz naturel, comprenant les étapes suivantes :
(a) mise à disposition du courant de départ contenant des hydrocarbures,
(b) envoi du courant de départ contenant des hydrocarbures à un étage d'oxydation partielle (POX),
(c) mise en réaction au moins partielle du courant de départ contenant des hydrocarbures dans l'étage d'oxydation partielle avec un courant d'un agent d'oxydation contenant de l'oxygène, dans des conditions d'oxydation partielle et en présence optionnelle d'un courant de modérateur, de préférence comprenant de la vapeur et/ou du dioxyde de carbone, conduisant à un courant de gaz de synthèse brut contenant de l'hydrogène (H₂), du monoxyde de carbone (CO), du dioxyde de carbone (CO₂) et du méthane (CH₄),
(d) évacuation du courant de gaz de synthèse brut hors de l'installation de production de gaz de synthèse et division du courant de gaz de synthèse brut en un premier courant partiel de gaz de synthèse brut et en un second courant partiel de gaz de synthèse brut,
(e) introduction d'au moins une partie du premier courant partiel de gaz de synthèse brut dans un réacteur de synthèse de méthanol, mise en réaction au moins partielle du premier courant partiel de gaz de synthèse brut dans le réacteur de synthèse de méthanol dans des conditions de synthèse de méthanol,
(f) évacuation hors du réacteur de synthèse de méthanol d'un premier courant de produit de réacteur contenant du méthanol, refroidissement du premier courant de produit de réacteur au-dessous de son point de rosée et fractionnement dans un dispositif de séparation de phases du premier courant de produit de réacteur, refroidi, en un premier courant de produit liquide et un premier courant de gaz résiduaire, qui contient des constituants de gaz de synthèse qui n'ont pas réagi et des composants inertes, évacuation hors du processus du premier courant de produit liquide, en tant que courant de produit-méthanol brut,
(g) division du premier courant de gaz résiduaire en un courant de lavage de synthèse de méthanol et en un courant de recyclage qui est renvoyé au réacteur de synthèse de méthanol,
(h) introduction d'au moins une partie du second courant de partiel de gaz de synthèse brut dans un dispositif de sorption destiné à l'élimination de dioxyde de carbone au moyen d'un procédé de sorption physique ou chimique, évacuation hors du dispositif de sorption d'un courant de gaz de synthèse appauvri en dioxyde de carbone et d'un courant de gaz enrichi en dioxyde de carbone,
(j) introduction d'au moins une partie du courant de gaz de synthèse appauvri en dioxyde de carbone dans un étage de séparation cryogénique de gaz, fractionnement dans l'étage de séparation cryogénique de gaz du courant de gaz de synthèse appauvri en dioxyde de carbone, en les courants partiels suivants :
(j1) un courant de gaz riche en monoxyde de carbone, qui est évacué hors du processus en tant que courant de produit-monoxyde de carbone,
(j2) un courant de gaz riche en hydrogène,
(j3) un courant de gaz résiduaire contenant des composants inertes, du méthane, de l'hydrogène et du monoxyde de carbone, qui est envoyé au moins en partie à un brûleur d'un dispositif de chauffage, en tant que premier gaz combustible,
**caractérisé en ce que** le courant de gaz enrichi en dioxyde de carbone est introduit au moins en partie dans le réacteur de synthèse de méthanol.

2. Procédé selon la revendication 1, **caractérisé en ce que** le courant de gaz riche en hydrogène est introduit au moins en partie dans le réacteur de synthèse de méthanol.

3. Procédé selon la revendication 2, **caractérisé en ce que** dans le réacteur de synthèse de méthanol est introduite une proportion du courant de gaz riche en hydrogène telle que le nombre stœchiométrique SN, qui se rapporte à la totalité de tous les courants de substances introduits dans le réacteur de synthèse de méthanol à l'entrée de réacteur du réacteur de synthèse de méthanol, se situe entre 1,8 et 2,4, de préférence entre 2,0 et 2,2.

4. Procédé selon la revendication 1 ou 2 ou 3, **caractérisé en ce que** le courant de gaz riche en hydrogène est envoyé au moins en partie au brûleur du dispositif de chauffage, en tant que second gaz combustible.

5. Procédé selon l'une quelconque des revendications précédentes, **caractérisé en ce qu'**une partie du courant de départ contenant des hydrocarbures est envoyée en tant que troisième gaz combustible au brûleur du dispositif de chauffage.

6. Procédé selon l'une quelconque des revendications précédentes, **caractérisé en ce qu'**au moins une partie du courant de lavage de synthèse de méthanol est envoyée en tant que quatrième gaz combustible au brûleur du dispositif de chauffage.

7. Procédé selon l'une quelconque des revendications précédentes, **caractérisé en ce que** le dispositif de chauffage sert à la production de vapeur, la vapeur produite servant au moins en partie de modérateur dans l'étage d'oxydation partielle.

8. Procédé selon l'une quelconque des revendications précédentes, **caractérisé en ce que** le dispositif de chauffage sert à la production de vapeur, la vapeur produite étant au moins en partie fournie à un utilisateur extérieur (vapeur exportée).

9. Procédé selon l'une quelconque des revendications précédentes, **caractérisé en ce qu'**en outre un courant de gaz contenant du dioxyde de carbone, qui provient d'une source extérieure au processus, est introduit dans le réacteur de synthèse de méthanol

10. Procédé selon l'une quelconque des revendications précédentes, **caractérisé en ce que** le dispositif de chauffage sert au préchauffage du courant de départ contenant des hydrocarbures et/ou du courant de l'agent d'oxydation contenant de l'oxygène.

11. Procédé selon l'une quelconque des revendications précédentes, **caractérisé en ce que** le courant de lavage de synthèse de méthanol est fractionné au moyen d'un dispositif de séparation, de préférence d'un dispositif de séparation sur membrane, en un premier courant de lavage enrichi en hydrogène et en un second courant de lavage appauvri en hydrogène et enrichi en oxydes de carbone et méthane, au moins une partie du premier courant de lavage enrichi en hydrogène étant envoyée au brûleur du dispositif de chauffage, en tant que quatrième gaz combustible, et au moins une partie du second courant de lavage enrichi en oxydes de carbone et méthane étant envoyée à l'étage d'oxydation partielle.

12. Installation destinée à la production de méthanol et monoxyde de carbone pur à partir d'un courant de départ contenant des hydrocarbures, de préférence de gaz naturel, comprenant les constituants suivants, se trouvant en connexion fluidique entre eux :
(a) des moyens destinés à la mise à disposition du courant de départ contenant des hydrocarbures,
(b) des moyens destinés à l'envoi du courant de départ contenant des hydrocarbures à un étage d'oxydation partielle (POX),
(c) un étage d'oxydation partielle, des moyens destinés à l'envoi d'un courant d'un agent d'oxydation contenant de l'oxygène à l'étage d'oxydation partielle, en option des moyens destinés à l'envoi d'un courant d'un courant de modérateur à l'étage d'oxydation partielle, des moyens destinés à l'évacuation hors de l'étage d'oxydation partielle d'un courant de gaz de synthèse brut contenant de l'hydrogène (H₂), du monoxyde de carbone (CO), du dioxyde de carbone (CO₂) ainsi que du méthane (CH₄),
(d) des moyens destinés à la division du courant de gaz de synthèse brut en un premier courant partiel de gaz de synthèse brut et en un second courant partiel de gaz de synthèse brut,
(e) un réacteur de synthèse de méthanol, des moyens destinés à l'introduction d'au moins une partie du premier courant partiel de gaz de synthèse brut dans le réacteur de synthèse de méthanol,
(f) des moyens destinés à l'évacuation hors du réacteur de synthèse de méthanol d'un premier courant de produit de réacteur contenant du méthanol, des moyens destinés au refroidissement du premier courant de produit de réacteur au-dessous de son point de rosée , un dispositif de séparation de phases destiné au fractionnement du premier courant de produit de réacteur, refroidi, en un premier courant de produit liquide et un premier courant de gaz résiduaire, qui contient des constituants de gaz de synthèse qui n'ont pas réagi et des composants inertes, des moyens destinés à l'évacuation hors du processus du premier courant de produit liquide, en tant que curant de produit-méthanol brut,
(g) des moyens destinés à la division du premier courant de gaz résiduaire en un courant de lavage de synthèse de méthanol et en un courant de recyclage qui est renvoyé au réacteur de synthèse de méthanol,
(h) un dispositif de sorption destiné à l'élimination de dioxyde de carbone au moyen d'un procédé de sorption physique ou chimique, un dispositif de chauffage comportant au moins un brûleur, des moyens destinés à l'introduction d'au moins une partie du second courant partiel de gaz de synthèse brut dans le dispositif de sorption, des moyens destinés à l'évacuation hors du dispositif de sorption d'un courant de gaz de synthèse appauvri en dioxyde de carbone et d'un courant de gaz enrichi en dioxyde de carbone,
(j) un étage de séparation cryogénique de gaz, approprié au fractionnement dans l'étage de séparation cryogénique de gaz du courant de gaz de synthèse appauvri en dioxyde de carbone, en les courants partiels suivants :
(j1) un courant de gaz riche en monoxyde de carbone, qui peut être évacué hors du processus en tant que courant de produit-monoxyde de carbone,
(j2) un courant de gaz riche en hydrogène,
(j3) un courant de gaz résiduaire contenant du méthane, de l'hydrogène et du monoxyde de carbone, qui peut être envoyé au moins en partie à au moins un brûleur du dispositif de chauffage, en tant que premier gaz combustible, des moyens destinés à l'introduction dans l'étage de séparation cryogénique de gaz d'au moins une partie du courant de gaz de synthèse appauvri en dioxyde de carbone
**caractérisé en ce que** sont en outre compris des moyens qui permettent au courant de gaz enrichi en dioxyde de carbone d'être introduit au moins en partie dans le réacteur de synthèse de méthanol.

13. Installation selon la revendication 12, **caractérisée en ce qu'**elle comprend en outre des moyens qui permettent au courant de gaz riche en hydrogène d'être envoyé au moins en partie au brûleur du dispositif de chauffage, en tant que second gaz combustible.

14. Installation selon l'une quelconque des revendications précédentes, **caractérisée en ce que** sont en outre compris des moyens qui permettent à une partie du courant de départ contenant des hydrocarbures, de préférence du gaz naturel, d'être envoyée en tant que troisième gaz combustible au brûleur du dispositif de chauffage.

15. Installation selon l'une quelconque des revendications précédentes, **caractérisée en ce que** sont en outre compris des moyens qui permettent à au moins une partie du courant de lavage de synthèse de méthanol d'être envoyée, en tant que quatrième gaz combustible, au brûleur du dispositif de chauffage.

16. Installation selon l'une quelconque des revendications précédentes, **caractérisée en ce que** sont en outre compris des moyens qui permettent au dispositif de chauffage de servir à la production de vapeur, la vapeur produite étant utilisable au moins en partie en tant que modérateur dans l'étage d'oxydation partielle.

17. Installation selon l'une quelconque des revendications précédentes, **caractérisée en ce que** sont en outre compris des moyens qui permettent au dispositif de chauffage de servir à la production de vapeur, la vapeur produite pouvant être fournie au moins en partie à un utilisateur extérieur (vapeur d'exportation)

18. Installation selon l'une quelconque des revendications précédentes, **caractérisée en ce que** sont en outre compris des moyens qui permettent à en outre un courant de gaz contenant du dioxyde de carbone, qui provient d'une source extérieure au processus, de pouvoir être introduit dans le réacteur de synthèse de méthanol.

19. Installation selon l'une quelconque des revendications précédentes, **caractérisée en ce que** sont en outre compris des moyens qui permettent au dispositif de chauffage d'être utilisable pour le préchauffage du courant de gaz de départ contenant des hydrocarbures et/ou du courant de l'agent d'oxydation contenant de l'oxygène.

20. Installation selon l'une quelconque des revendications précédentes, **caractérisée en ce que** sont en outre compris des moyens qui permettent
- au courant de lavage de synthèse de méthanol de pouvoir être fractionné au moyen d'un dispositif de séparation, de préférence d'un dispositif de séparation sur membrane, en un premier courant de lavage enrichi en hydrogène et en un second courant de lavage appauvri en hydrogène et enrichi en oxydes de carbone et méthane,
- à au moins une partie du premier courant de lavage enrichi en hydrogène de pouvoir être envoyée, en tant que quatrième gaz combustible, au brûleur du dispositif de chauffage.
- à au moins une partie du second courant de lavage enrichi en oxydes de carbone et méthane de pouvoir être envoyée à l'étage d'oxydation partielle.
